(19) Europäisches Patentamt
European Patent Office
Office européen des brevets

(11) Veröffentlichungsnummer: **0 173 315**
**A1**

(12)

# EUROPÄISCHE PATENTANMELDUNG

(21) Anmeldenummer: **85110826.6**

(22) Anmeldetag: **19.08.85**

(51) Int. Cl.[4]: **C 07 D 239/42**, A 01 N 47/44

(30) Priorität: **30.08.84 DE 3431913**

(43) Veröffentlichungstag der Anmeldung: **05.03.86 Patentblatt 86/10**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Diehr, Hans-Joachim, Dr., Hoehe 35, D-5600 Wuppertal 1 (DE)**
Erfinder: **Fest, Christa, Dr., Im Johannistal 20, D-5600 Wuppertal 1 (DE)**

(72) Erfinder: **Kirsten, Rolf, Dr., Carl-Langhans-Strasse 27, D-4019 Monheim (DE)**
Erfinder: **Kluth, Joachim, Dr., Kurt-Schumacher-Strasse 9, D-4018 Langenfeld (DE)**
Erfinder: **Müller, Klaus-Helmut, Dr., Bockhackstrasse 55, D-4000 Düsseldorf (DE)**
Erfinder: **Pfister, Theodor, Dr., Lichtenberger Strasse 30, D-4019 Monheim (DE)**
Erfinder: **Priesnitz, Uwe, Dr., Severinstrasse 58, D-5650 Solingen 1 (DE)**
Erfinder: **Riebel, Hans-Joachem, Dr., In der Beek 92, D-5600 Wuppertal 1 (DE)**
Erfinder: **Roy, Wolfgang, Dr., Walter-Kolb-Strasse 47, D-4018 Langenfeld (DE)**
Erfinder: **Eue, Ludwig, Dr., Paul-Klee-Strasse 36, D-5090 Leverkussen (DE)**
Erfinder: **Santel, Hans-Joachim, Dr., Gerstenkamp 19, D-5000 Koeln 80 (DE)**
Erfinder: **Schmidt, Robert R., Dr., Im Waldwinkel 110, D-5060 Bergisch-Gladbach 2 (DE)**

(54) **Oxyguanidin-Derivate.**

(57) Die Erfindung betrifft neue Oxyguanidin-Derivate der allgemeinen Formel (I)

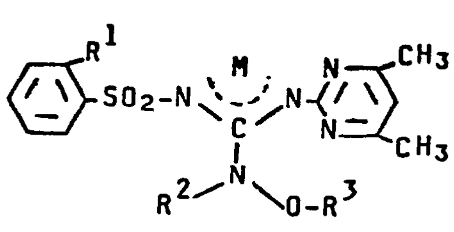

(I)

in welcher
M für Wasserstoff oder ein Metalläquivalent steht,
$R^1$ für Halogen, $C_1$–$c_4$-Alkyl, $C_1$–$C_2$-Halogenalkyl, $C_1$–$C_4$-Alkoxy, Phenyl oder $C_1$–$C_2$-Alkoxy-carbonyl steht,
$R^2$ für Wasserstoff oder für den Rest 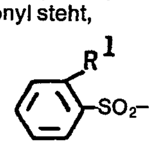
steht, worin $R^1$ die oben angegebene Bedeutung hat, und
$R^3$ für $C_4$–$C_{12}$-Alkyl, Isopropyl oder – für den Fall, dass M für ein Metalläquivalent steht – auch Propyl, für $C_2$–$C_{12}$-Halogenalkyl, für $C_1$–$C_4$-Alkoxy-carbonyl-$C_1$–$C_2$-alkyl, für Aminocarbonylmethyl, $C_1$–$C_4$-Alkyl-aminocarbonylmethyl oder Di-$C_1$–$C_4$-Alkyl-amino-carbonylmethyl, für $C_3$–$C_{12}$-Alkenyl, für $C_3$–$C_{12}$-Alkinyl, für Carboxy-$C_1$–$C_2$-alkyl, für durch Halogen, Nitro, Cyano, $C_1$–$C_4$-Alkyl, $C_1$–$C_4$-Alkoxy oder $C_1$–$C_4$-Alkoxy-carbonyl substituiertes Benzyl, oder für gegebenenfalls durch Halogen, Nitro, Cyano, $C_1$–$C_4$-Alkyl, $C_1$–$C_4$-Alkoxy oder $C_1$–$C_4$-Alkoxycarbonyl substituiertes Phenyl, Phenylethyl oder Benzhydryl steht, sowie 1:1-Addukte von Verbindungen der Formel (I) mit starken Säuren, mehrere Verfahren und neue Zwischenprodukte zu ihrer Herstellung und ihre Verwendung als Herbizide.

EP 0 173 315 A1

0 173 315

BAYER AKTIENGESELLSCHAFT
Konzernverwaltung RP
Patentabteilung

5090 Leverkusen, Bayerwerk
Bi/mö
Ib

Oxyguanidin-Derivate

Die Erfindung betrifft neue Oxyguanidin-Derivate, mehrere Verfahren und neue Zwischenprodukte zu ihrer Herstellung und ihre Verwendung als Herbizide.

Verschiedene Guanidin-Derivate sind aus Patentschriften (vergl. DE-AS 1 089 210, DD-PS 71 016 und 84 530) als potentielle Herbizide bekannt geworden, haben jedoch bisher als Mittel zur Unkrautbekämpfung und/oder Regulierung des Pflanzenwachstums keine größere Bedeutung erlangt.
Weitere Guanidin-Derivate sind Gegenstand einer nicht zum vorveröffentlichten Stand der Technik gehörenden Patentanmeldung der Anmelderin (vergl. DE-OS 3 334 455), vgl. ferner EP-A- 117 014.
Es wurden nun neue Oxyguanidin-Derivate der allgemeinen Formel (I)

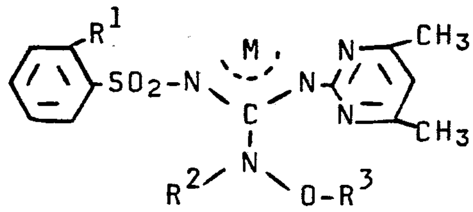

(I)

in welcher

M für Wasserstoff oder ein Metalläquivalent steht,

$R^1$ für Halogen, $C_1$-$C_4$-Alkyl, $C_1$-$C_2$-Halogenalkyl, $C_1$-$C_4$-Alkoxy, Phenyl oder $C_1$-$C_2$-Alkoxy-carbonyl steht,

$R^2$ für Wasserstoff oder für den Rest 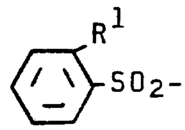 steht, worin $R^1$ die oben angegebene Bedeutung hat, und

$R^3$ für $C_4$-$C_{12}$-Alkyl, Isopropyl oder - für den Fall, daß M für ein Metalläquivalent steht - auch Propyl, für $C_2$-$C_{12}$-Halogenalkyl, für $C_1$-$C_4$-Alkoxy-carbonyl-$C_1$-$C_2$-alkyl, für Aminocarbonylmethyl, $C_1$-$C_4$-Alkyl-amino-carbonylmethyl oder Di-$C_1$-$C_4$-Alkyl-amino-carbonyl-methyl, für $C_3$-$C_{12}$-Alkenyl, für $C_3$-$C_{12}$-Alkinyl, für Carboxy-$C_1$-$C_2$-alkyl, für durch Halogen, Nitro, Cyano, $C_1$-$C_4$-Alkyl, $C_1$-$C_4$-Alkoxy oder $C_1$-$C_4$-Alkoxy-carbonyl substituiertes Benzyl, oder für gegebenenfalls durch Halogen, Nitro, Cyano, $C_1$-$C_4$-Alkyl, $C_1$-$C_4$-Alkoxy oder $C_1$-$C_4$-Alkoxycarbonyl substituiertes Phenyl, Phenylethyl oder Benzhydryl steht,

sowie 1:1-Addukte von Verbindungen der Formel (I) mit starken Säuren gefunden.

Die allgemeine Formel (I) steht - wenn M für Wasserstoff steht - für die einzelnen Tautomeren der Formeln (IA) und (IB)

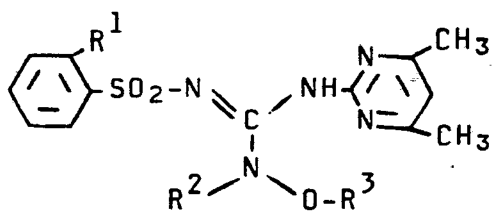

(IA)

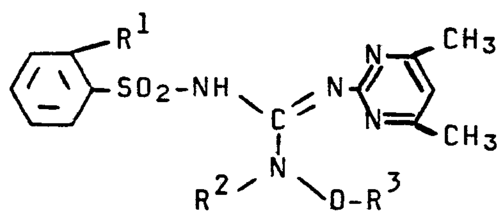

(IB)

in welcher

$R^1$, $R^2$ und $R^3$ die oben angegebenen Bedeutungen haben,

sowie für Gemische der Tautomeren (IA) und (IB).

Das Mischungsverhältnis (IA)/(IB) hängt von aggregationsbestimmenden Faktoren, wie z. B. Temperatur, Lösungsmittel und Konzentration ab.

Für den Fall, daß neben M auch $R^2$ für Wasserstoff steht, ist eine weitere tautomere Form (IC) möglich:

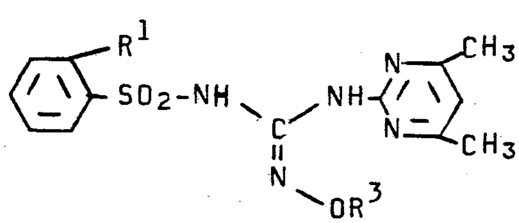

(IC)

Alle diese Tautomeren werden im Rahmen der vorliegenden Erfindung beansprucht.

Man erhält die neuen Oxyguanidin-Derivate der Formel (I), wenn man

(a) für den Fall, daß $R^2$ für den Rest 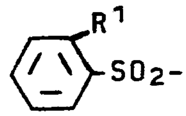 und M für Wasserstoff steht, 4,6-Dimethyl-2-oxyguanidino-pyrimidin-Derivate der Formel (II)

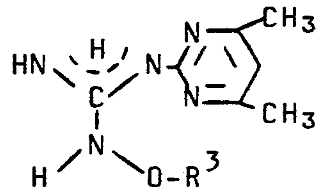

(II)

in welcher

$R^3$ die oben angegeben Bedeutung hat,

mit wenigstens zwei Moläquivalenten von Arensulfonsäurechloriden der Formel (III)

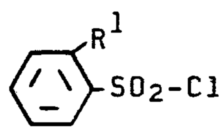

(III)

in welcher

$R^1$ die oben angegebene Bedeutung hat,

in Gegenwart von Säureakzeptoren und gegebenenfalls in Gegenwart von Verdünnungsmitteln umsetzt;

oder

(b) für den Fall, daß $R^2$ für Wasserstoff steht, die nach dem unter (a) angegebenen Verfahren erhältlichen Oxyguanidin-Derivate der Formel (ID)

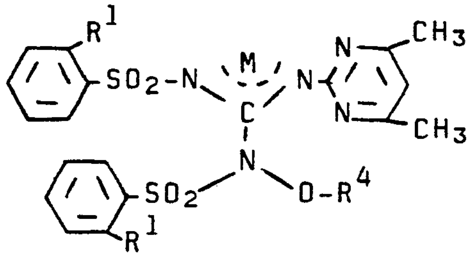

(ID)

in welcher

M und $R^1$ die oben angegebenen Bedeutungen haben und

$R^4$ die oben für $R^3$ angegebene Bedeutung haben kann oder für Methyl oder Ethyl steht,

mit Hydroxylamin-Derivaten der Formel (IV)

$$H_2N-O-R^3 \quad (IV)$$

in welcher

$R^3$ die oben angegebene Bedeutung hat,

oder mit Hydrochloriden von Verbindungen der Formel (IV),

gegebenenfalls in Gegenwart von Säureakzeptoren und gegebenenfalls in Gegenwart von Verdünnungsmitteln umsetzt;

oder

(c) für den Fall, daß M für ein Metalläquivalent steht, die nach den oben unter (a) und (b) angegebenen Verfahren erhältlichen Verbindungen der Formel (I), in welcher M für Wasserstoff steht und $R^1$, $R^2$ und $R^3$ die oben angegebenen Bedeutungen haben,

mit Metall-hydroxiden, -hydriden oder -alkanolaten oder mit metallorganischen Verbindungen, gegebenenfalls in Gegenwart von Verdünnungsmitteln umsetzt;

oder

(d) für den Fall, daß 1 : 1-Addukte von Verbindungen der Formel (I) mit starken Säuren herzustellen sind,

Oxyguanidin-Derivate der Formel (I), in welcher M für Wasserstoff steht und $R^1$, $R^2$ und $R^3$ die oben angegebenen Bedeutungen haben,

mit starken Säuren, gegebenenfalls in Gegenwart von inerten Verdünnungsmitteln umsetzt.

Die neuen Oxyguanidin-Derivate der Formel (I) und ihre 1 : 1-Addukte mit starken Säuren zeichnen sich durch starke herbizide Wirksamkeit aus.

Ueberraschenderweise zeigen die neuen Oxyguanidin-Derivate der Formel (I) und ihre 1 : 1-Addukte eine wesentlich bessere herbizide Wirksamkeit als vorbekannte Guanidine gleicher Wirkungsrichtung.

Gegenstand der Erfindung sind vorzugsweise Verbindungen der Formel (I), in welcher

M für Wasserstoff oder ein Natrium-, Kalium- oder Calcium-äquivalent steht,

$R^1$ für Fluor, Chlor, Brom, Methyl, Chlormethyl, Trifluormethyl, Methoxy, Phenyl oder Methoxycarbonyl steht,

$R^2$ für Wasserstoff oder den Rest 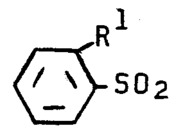 steht,

worin

$R^1$ für Fluor, Chlor, Brom, Methyl, Trifluormethyl, Methoxy, Phenyl, Methoxycarbonyl oder Ethoxycarbonyl steht, und

$R^3$ für $C_4$-$C_8$-Alkyl, Isopropyl oder - für den Fall, daß M für ein Metalläquivalent steht - auch Propyl, für $C_2$-$C_8$-Halogenalkyl, für$C_1$-$C_2$-Alkoxycarbonyl-$C_1$-$C_2$-alkyl, für Amino-carbonylmethyl, $C_1$-$C_3$-Alkyl-amino-carbonylmethyl oder Di-$C_1$-$C_3$-alkyl-aminocarbonylmethyl, für $C_3$-$C_8$-Alkenyl, für $C_3$-$C_8$-Alkinyl, für durch Fluor, Chlor, Nitro, Cyano, Methyl, Methoxy oder $C_1$-$C_2$-Alkoxy-carbonyl substituiertes Benzyl, oder für gegebenenfalls durch Fluor, Chlor, Nitro, Cyano, Methyl, Methoxy oder $C_1$-$C_2$-Alkoxy-carbonyl substituiertes Phenyl, Phenylethyl oder Benzhydryl steht.

Gegenstand der Erfindung sind vorzugsweise ferner 1 : 1-Addukte von Verbindungen der Formel (I) - wie vorausgehend definiert - mit Halogenwasserstoffsäuren, wie Hydrogenfluorid, -chlorid, -bromid und -iodid, mit Schwefelsäure, Trifluoressigsäure, mit gegebenenfalls durch Fluor oder

Chlor substituierten Alkansulfonsäuren mit bis zu 4 Kohlenstoffatomen oder auch mit Benzol- oder Naphthalinsulfonsäuren, welche gegebenenfalls durch Fluor, Chlor, Brom oder Methyl substituiert sind.

Gegenstand der Erfindung sind insbesondere Verbindungen der Formel (I), in welcher

M für Wasserstoff oder ein Natrium-, Kalium- oder Calcium-äquivalent steht,

$R^1$ für Fluor, Chlor, Brom, Chlormethyl oder Methoxycarbonyl steht,

$R^2$ für den Rest 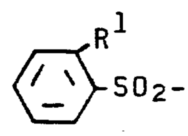 steht,

worin

$R^1$ für Fluor, Chlor, Brom oder Methoxycarbonyl steht, und

$R^3$ für $C_4$-$C_8$-Alkyl, Isopropyl oder - für den Fall, daß M für ein Metalläquivalent steht - auch Propyl, für $C_2$-$C_4$-Halogenalkyl, für $C_1$-$C_2$-Alkoxycarbonyl-$C_1$-$C_2$-alkyl, für Aminocarbonylmethyl, für $C_3$-$C_5$-Alkenyl, für $C_3$-$C_5$-Alkinyl, für durch Fluor, Chlor, Nitro, Methyl oder $C_1$-$C_2$-Alkoxycarbonyl substituiertes Benzyl, oder für gegebenenfalls durch Fluor, Chlor, Nitro, Methyl oder $C_1$-$C_2$-Alkoxy-carbonyl substituiertes Phenyl, Phenylethyl oder Benzhydryl steht,

sowie - für den Fall, daß M für Wasserstoff steht - die 1 : 1-Addukte der vorausgehend definierten Verbindungen der Formel (I) mit Salzsäure, Schwefelsäure, Trifluoressigsäure, Methansulfonsäure, Benzolsulfonsäure und p-Toluolsulfonsäure.

Verwendet man beispielsweise für die Verfahrensvariante (a) 2-Fluor-benzolsulfonsäurechlorid und N'-(4,6-Dimethyl-pyrimidin-2-yl)-N''-isopropoxy-guanidin als Ausgangsstoffe so kann der Reaktionsablauf durch folgendes Formelschema skizziert werden:

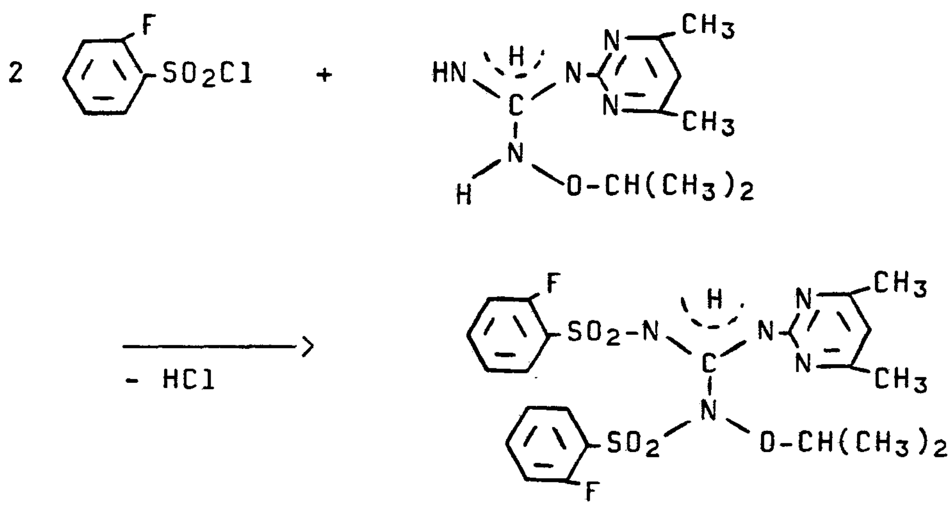


Verwendet man beispielsweise für die Verfahrensvariante (b) N'-(4,6-Dimethyl-pyrimidin-2-yl)-N''-methoxy-N'',N'''-bis-(2-brom-benzolsulfonyl)-guanidin und O-Hexylhydroxylamin als Ausgangsstoffe, so kann der Reaktionsablauf durch folgendes Formelschema skizziert werden:

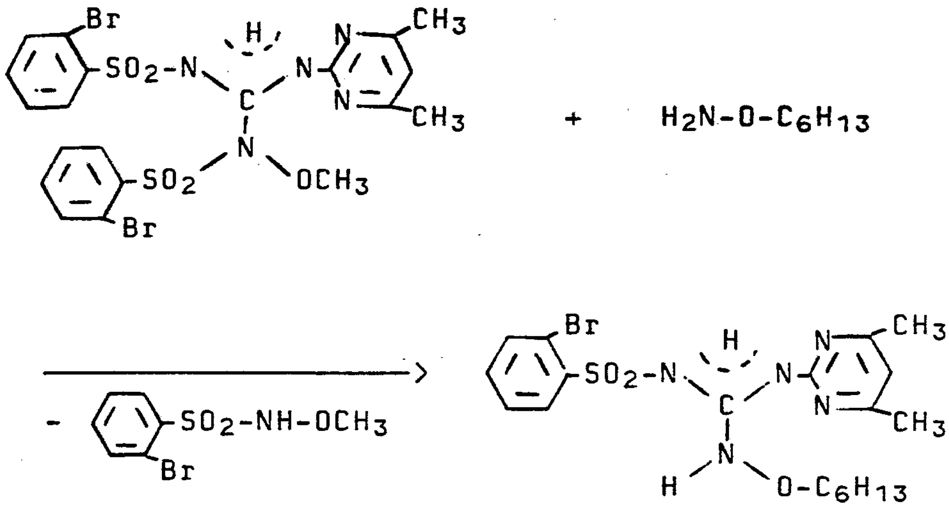

Verwendet man beispielsweise für die Verfahrensvariante (c) N'-(4,6-Dimethyl-pyrimidin-2-yl)-N''-phenoxy-N'''-(2-chlor-benzolsulfonyl)-guanidin und Kaliumethanolat als Ausgangsstoffe, so kann der Reaktionsablauf durch das folgende Formelschema skizziert werden:

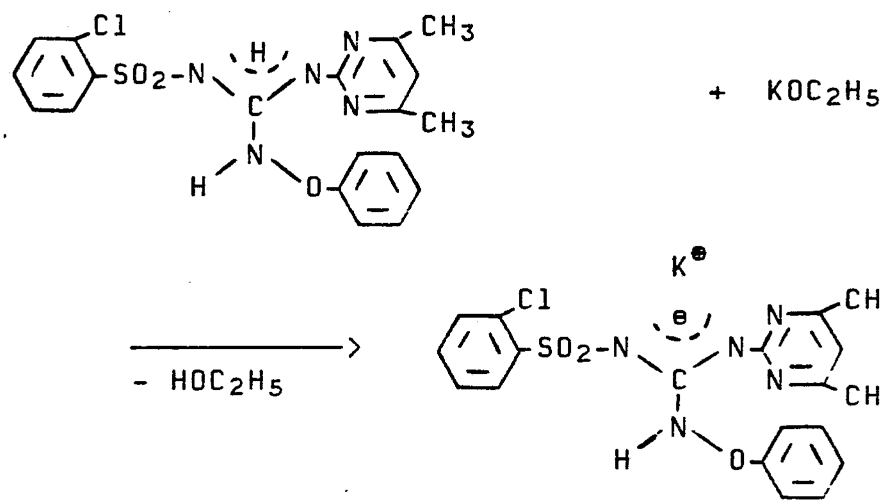


Verwendet man beispielsweise für die Verfahrensvariante (d) N'-(4,6-Dimethyl-pyrimidin-2-yl)-N''-pentoxy-N'',N'''-bis-(2-methoxycarbonylbenzolsulfonyl)-guanidin und Salzsäure als Ausgangsstoffe, so kann der Reaktionsablauf durch folgendes Formelschema skizziert werden:

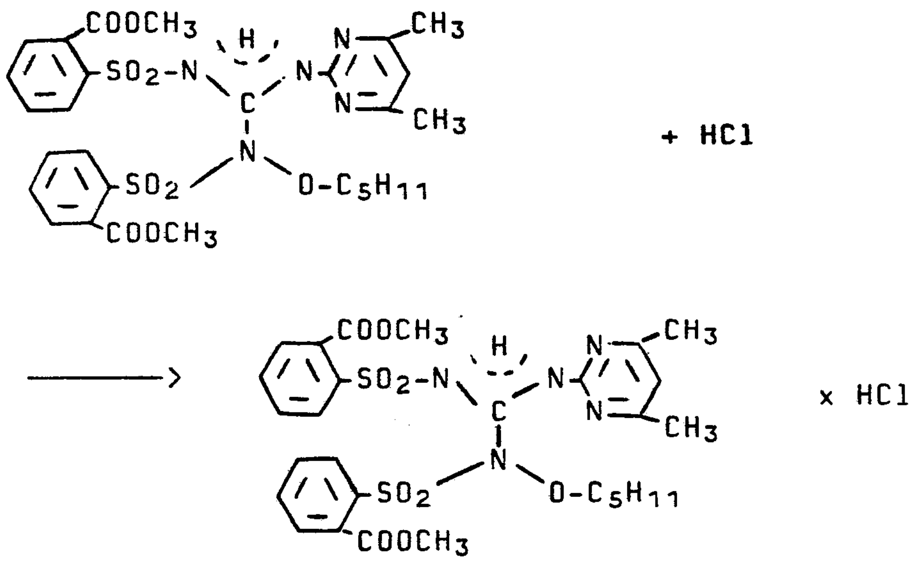


Die als Ausgangsstoffe für die Verfahrensvariante (a) zu verwendenden 4,6-Dimethyl-2-oxyguanidino-pyrimidin-Derivate sind durch die Formel (II) allgemein definiert. In Formel (II) hat $R^3$ vorzugsweise bzw. insbesondere bevorzugt die gleiche Bedeutung, wie sie oben im Rahmen der Substituentendefinition der Formel (I) vorzugsweise bzw. als insbesondere bevorzugt angegeben ist.

Als Ausgangsstoffe der Formel (II) seien beispielsweise genannt:

N'-(4,6-Dimethyl-pyrimidin-2-yl)-N''-propoxy-guanidin, -N''-isopropoxy-guanidin, -N''-butoxy-guanidin, -N''-isobutoxy-guanidin, -N''-sec.-butoxy-guanidin, -N''-pentoxy-guanidin, -N''-isopentoxy-guanidin, -N''-sec.-pentoxy-guanidin, -N''-hexyloxy-guanidin, -N''-isohexyloxy-guanidin, -N''-heptyloxy-guanidin, -N''-isoheptyloxy-guanidin, -N''-octyloxy-guanidin, -N''-isooctyloxy-guanidin,

-N''-allyloxy-guanidin, -N''-crotyloxy-guanidin, -N''-(2-chlor-ethoxy)-guanidin, -N''-(2-fluor-ethoxy)-guanidin, -N''-(2-chlor-propoxy)-guanidin, -N''-(3-chlor-propoxy)-guanidin, -N''-(4-chlor-butoxy)-guanidin, -N''-methoxy-carbonylmethyloxy-guanidin, -N''-ethoxycarbonylmethoxy-guanidin, -N''-(1-methoxycarbonyl-ethoxy)-guanidin, -N''-(1-ethoxy-carbonyl-ethoxy)-guanidin, -N''-aminocarbonylmethoxy-guanidin, -N''-(phenyl-ethoxy)-guanidin, -N''-phenoxy-guanidin, -N''-(4-methyl-benzyloxy)-guanidin, -N''-(4-chlor-benzyloxy)-guanidin, -N''-(4-nitro-benzyl-oxy)-guanidin, -N''-(2,6-dichlor-benzyloxy)-guanidin, -N''(4-ethoxy-carbonyl-benzyloxy)-guanidin und -N''-(4-methoxycarbonyl-benzyloxy)-guanidin.

Die Ausgangsstoffe der Formel (II) sind teilweise bekannt (vergl. J. Chem. Soc. 1962, 3915); zum Teil sind sie Gegenstand einer nicht zum vorveröffentlichten Stand der Technik gehörenden Patentanmeldung der Anmelderin (vergl. DE-OS 3 334 455).

Man erhält die neuen Verbindungen der Formel (IIa)

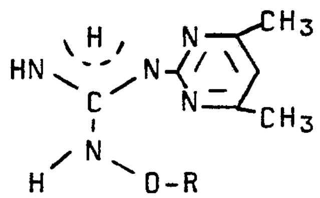

(IIa)

in welcher

R für $C_2$-$C_{12}$-Halogenalkyl, für $C_1$-$C_4$-Alkoxy-carbonyl-$C_1$-$C_2$-alkyl, für Aminocarbonylmethyl, $C_1$-$C_4$-Alkyl-amino-carbonylmethyl oder Di-$C_1$-$C_4$-alkyl-amino-carbonylmethyl,

für $C_4$-$C_{12}$-Alkenyl, für $C_4$-$C_{12}$-Alkinyl, für durch Halogen, Nitro, Cyano, $C_1$-$C_4$-Alkyl, $C_1$-$C_4$-Alkoxy oder $C_1$-$C_4$-Alkoxy-carbonyl substituiertes Benzyl oder für gegebenenfalls durch Halogen, Nitro, Cyano, $C_1$-$C_4$-Alkyl, $C_1$-$C_4$-Alkoxy oder $C_1$-$C_4$-Alkoxy-carbonyl substituiertes Phenyl oder Phenylethyl steht,

wenn man 2-Cyanamino-4,6-dimethyl-pyrimidin der Formel (V)

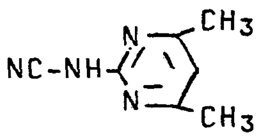

(V)

mit Hydroxylamin-Derivaten der Formel (IVa)

$$H_2N\text{-}O\text{-}R \qquad \text{(IVa)}$$

in welcher

R die oben angegebene Bedeutung hat,

oder mit deren Hydrochloriden,

gegebenenfalls in Gegenwart von Verdünnungsmitteln, wie z. B. Ethanol, Isopropanol oder Butanol, bei Temperaturen zwischen 20 °C und 150 °C, vorzugsweise zwischen 50 °C und 120 °C, umsetzt und gegebenenfalls die Umsetzungsprodukte mit Säureakzeptoren, wie z. B. Ammoniak, Natronlauge oder Soda, behandelt. - Auch die Verbindungen der Formel (II) können in Form verschiedener Tautomeren vorliegen; diese sind auch Gegenstand der Erfindung.

2-Cyanamino-4,6-dimethyl-pyrimidin der Formel (V) ist bereits bekannt (vgl. J. Chem. Soc. 1953, 1725).

Hydroxylamin-Derivate der Formel (IVa) sind ebenfalls bereits bekannt und können nach an sich bekannten Verfahren hergestellt werden (vergl. Chem. Pharm. Bull. 15 (1967), 345; Bull. Soc. Chim. France 1958, 664; Synthesis 1976, 682; J. Chem. Soc. 1930, 228 und Helv. Chim. Acta 45 (1962), 1387).

Die weiter als Ausgangsstoffe für die Verfahrensvariante (a) zu verwendenden Arensulfonsäurechloride sind durch die Formel (III) allgemein definiert. In Formel (III) hat $R^1$ vorzugsweise bzw. insbesondere bevorzugt die gleiche Bedeutung, wie sie oben im Rahmen der Substituentendefinition der Formel (I) vorzugsweise bzw. insbesondere bevorzugt angegeben ist.

Als Ausgangsstoffe der Formel (III) seien beispielsweise genannt:

2-Chlor-, 2-Fluor-, 2-Brom-, 2-Methyl-, 2-Trifluormethyl-, 2-Methoxy-, 2-Phenyl- und 2-Methoxycarbonyl-benzolsulfonsäurechlorid.

Die Arensulfonsäurechloride der Formel (III) sind bekannt und können nach an sich bekannten Verfahren hergestellt werden (vergl. J. Org. Chem. 33 (1968), 2104; J. Org. Chem. 25 (1960), 1824 und DE-OS 2 308 262).

Die als Ausgangsstoffe für die Verfahrensvarante (b) zu verwendenden Oxyguanidin-Derivate sind durch die Formel (ID) allgemein definiert. In Formel (ID) haben M und $R^1$ vorzugsweise bzw. insbesondere bevorzugt die gleichen Bedeutungen, wie sie oben im Rahmen der Substituentendefinition der

Formel (I) vorzugsweise bzw. als insbesondere bevorzugt angegeben sind und $R^4$ steht vorzugsweise für Methyl.

Als Beispiele für die als Ausgangsstoffe in Verfahren (b) zu verwendenden Verbindungen der Formel (ID) seien genannt:

N'-(4,6-Dimethyl-pyrimidin-2-yl)-N''-methoxy-
-N'',N'''-bis-(2-chlor-benzolsulfonyl)-,
-N'',N'''-bis-(2-brom-benzolsulfonyl)-,
-N'',N'''-bis-(2-fluor-benzolsulfonyl)-,
-N'',N'''-bis-(2-methyl-benzolsulfonyl)-,
-N'',N'''-bis-(2-trifluormethyl-benzolsulfonyl)-,
-N'',N'''-bis-(2-methoxy-benzolsulfonyl)-,
-N'',N'''-bis-(2-phenyl-benzolsulfonyl)- und
-N'',N'''-bis-(2-methoxycarbonyl-benzolsulfonyl)-
-guanidin.

Die Guanidin-Derivate der Formel (ID) können nach dem oben unter (a) beschriebenen Verfahren hergestellt werden.

Die bei der Verfahrensvariante (b) weiter als Ausgangsstoffe zu verwendenden Hydroxylamin-Derivate sind durch die Formel (IV) allgemein definiert. In der Formel (IV) hat $R^3$ vorzugsweise bzw. insbesondere bevorzugt die gleiche Bedeutung, wie sie oben im Rahmen der Substituentendefinition der Formel (I) vorzugsweise bzw. als insbesondere bevorzugt angegeben ist.

Als Ausgangsstoffe der Formel (IV) seien beispielsweise genannt:

O-Propyl-, O-Isopropyl-, O-Butyl-, O-Isobutyl-, O-sec.-Butyl-, O-Pentyl-, O-Isopentyl-, O-sec.-Pentyl-, O-Hexyl-, O-Isohexyl-, O-Heptyl-, O-Isoheptyl-, O-Octyl-, O-Isooctyl-, O-Allyl-, O-Crotyl-, O-(2-Chlor-ethyl)-, O-(2-Fluorethyl)-, O-(2-Chlor-propyl)-, O-(3-Chlor-propyl)-, O-(4-Chlor-butyl)-, O-Methoxycarbonylmethyl-, O-Ethoxycarbonylmethyl-, O-(1-Methoxycarbonyl)-ethyl-, O-(1-Ethoxycarbonyl)-ethyl-, O-Aminocarbonylmethyl-, O-(2-Phenyl-ethyl)-, O-Phenyl-, O-(4-Methyl-benzyl)-, O-(4-Fluor-benzyl)-, O-(4-Chlor-benzyl)-, O-(4-Nitro-benzyl)-, O-(2,6-Dichlorbenzyl)-, O-(4-Methoxycarbonyl-benzyl)- und O-(4-Ethoxycarbonyl-benzyl)-hydroxylamin.

Hydroxylamin-Derivate der Formel (IV) sind bekannt und können nach an sich bekannten Verfahren hergestellt werden (vergl. Chem. Pharm. Bull. 15 (1967), 345; Bull. Soc. Chim. France 1958, 664; Synthesis 1976, 682; J. Chem. Soc. 1930, 228 und Helv. Chim. Acta 45 (1962), 1387).

Die bei der Verfahrensvariante (c) als Ausgangsstoffe zu verwendenden Oxyguanidin-Derivate sind durch die Formel (I) - mit der Maßgabe, daß M für Wasserstoff steht - allgemein definiert. In Formel (I) - soweit sie die als Ausgangsstoffe für Verfahren (c) zu verwendenden Verbindungen betrifft - steht M für Wasserstoff und die Reste $R^1$, $R^2$ und $R^3$ haben vorzugsweise bzw. insbesondere bevorzugt die die gleichen Bedeutungen, wie sie oben im Rahmen der Substituentendefinition für Formel (I) vorzugsweise bzw. als besonders bevorzugt angegeben sind.

Die als Ausgangsstoffe für Verfahren (c) zu verwendenden Verbindungen der Formel (I) können nach den unter (a) und

(b) beschriebenen Verfahren hergestellt werden.

Als Beispiele für die bei Verfahren (c) zu verwendenden Metallhydroxide, -hydride, -alkanolate bzw. metallorganischen Verbindungen seien genannt:

Lithium-, Natrium-, Kalium-, Magnesium- und Calcium-hydroxid, Lithium-, Natrium-, und Calcium-hydrid, Natriummethanolat und -ethanolat, Kalium-methanolat, -ethanolat, und Kalium-tert.-butanolat sowie Butyllithium und Isopropylmagnesiumchlorid.

Die bei der Verfahrensvariante (d) als Ausgangsstoffe zu verwendenden Oxyguanidin-Derivate sind durch die Formel (I) - mit der Maßgabe, daß M für Wasserstoff steht - allgemein definiert. In Formel (I) - soweit sie die als Ausgangsstoffe für Verfahren (d) zu verwendenden Verbindungen betrifft - steht M für Wasserstoff und die Reste $R^1$, $R^2$ und $R^3$ haben vorzugsweise bzw. insbesondere bevorzugt die gleichen Bedeutungen, wie sie oben im Rahmen der Substituentendefinition für Formel (I) vorzugsweise bzw. als insbesondere bevorzugt angegeben sind.

Die als Ausgangsstoffe für Verfahren (d) zu verwendenden Verbindungen der Formel (I) können nach den unter (a) und (b) beschriebenen Verfahren hergestellt werden.

Bei Verfahren (d) werden starke Säuren als Ausgangsstoffe eingesetzt. Vorzugsweise sind dies Halogenwasserstoffsäuren, wie Hydrogenfluorid, Hydrogenchlorid, Hydrogenbromid und Hydrogeniodid, weiter Schwefelsäure und Phosphorsäure oder gegebenenfalls durch Fluor oder Chlor substituierte Alkansulfonsäuren mit bis zu 4 Kohlenstoffatomen, wie z. B. Methansulfonsäure, Ethansulfonsäure, Chlormethansulfonsäure, 2-Chlorethansulfonsäure und Trifluormethansulfonsäure, ferner Benzolsulfonsäure, p-Toluolsulfonsäure, Naphthlin-1-sulfonsäure, Naphthalin-2-sulfonsäure, Naphthalin-1,4-, -1,5-, -1,6-, -2,6- und -2,7-disulfonsäure. Insbesondere bevorzugt sind Salzsäure (Hydrogenchlorid), Schwefelsäure, Benzolsulfonsäure und p-Toluolsulfonsäure.

Das erfindungsgemäße Verfahren (a) zur Herstellung der neuen Verbindungen der Formel (I) wird vorzugsweise unter Verwendung von Verdünnungsmitteln durchgeführt. Als solche kommen praktisch alle inerten organischen, vorzugsweise jedoch aprotisch polare Solventien in Betracht. Hierzu gehören gegebenenfalls halogenierte Kohlenwasserstoffe, wie z. B. Methylenchlorid, Chloroform, Toluol und Chlorbenzol, Nitrile, wie z. B. Acetonitril und Propionsäurenitril, Dimethylformamid, Dimethylacetamid, Dimethylsulfoxid, Sulfolan, Hexamethylphosphorsäuretriamid, 1,2-Dimethoxyethan, Pyridin und 2-Methyl-5-ethyl-pyridin.

Als Säureakzeptoren können bei Verfahren (a) praktisch alle üblicherweise verwendeten Säurebindemittel eingesetzt werden. Hierzu gehören insbesondere Alkalimetall- und Erdalkalimetall-hydride, metallorganische Verbindungen, wie Butyllithium, ferner aliphatische, aromatische oder heterocyclische Amine, wie Trimethylamin, Triethylamin, N,N-Dimethylanilin, N,N-Dimethyl-benzylamin, Diazabicyclooctan (DABCO), Diazabicycloundecen (DBU), Pyridin, 2-Methyl-5-ethyl-pyridin und 4-Dimethylamino-pyridin.

Die Reaktionstemperaturen können bei Verfahren (a) innerhalb eines größeren Bereiches variiert werden. Im allgemeinen arbeitet man zwischen -80 °C und +100 °C, vorzugsweise zwischen -30 °C und +50 °C. Das erfindungsgemäße Verfahren wird im allgemeinen bei Normaldruck durchgeführt.

Zur Durchführung von Verfahren (a) setzt man je Mol Verbindung der Formel (II) im allgemeinen zwischen 2 und 5 Mol, vorzugsweise zwischen 2,1 und 3 Mol Arensulfonsäurechlorid der Formel (III) ein. Die Reaktionskomponenten werden gewöhnlich bei Raumtemperatur oder unter Außenkühlung zusammengegeben und das Reaktionsgemisch wird bis zum Reaktionsende gerührt.

Die Aufarbeitung und Isolierung der neuen Verbindungen erfolgt nach üblichen Methoden : Gegebenenfalls nach Abdestillieren flüchtiger Komponenten wird mit Wasser und einem mit Wasser nicht mischbaren Lösungsmittel, wie z.B. Methylenchlorid, Chloroform oder Toluol, geschüttelt, die organische Phase mit Wasser gewaschen, getrocknet, filtriert und eingeengt. Die im Rückstand verbleibenden Produkte der Formel (I) werden durch Digerieren mit organischen Lösungsmitteln, wie z.B. Diethylether, Essigester, Ethanol oder Isopropanol zur Kristallisation gebracht und gegebenenfalls durch Umkristallisieren gereinigt.

Das erfindungsgemäße Verfahren (b) wird vorzugsweise unter Verwendung von Verdünnungsmitteln durchgeführt. Als solche kommen praktisch alle inerten organischen Lösungsmittel und gegebenenfalls zusätzlich auch Wasser in Betracht. Hierzu gehören insbesondere Alkohole, wie Methanol, Ethanol, n- und iso-Propanol, Ether, wie Tetrahydrofuran, Dioxan und 1,2-Dimethoxyethan, Ester,

wie Essigsäuremethylester und -ethylester, Nitrile, wie z. B. Acetonitril oder Propionsäurenitril, sowie Dimethylformamid und Wasser.

Als Säureakzeptoren können bei Verfahren (b) Säurebindemittel eingesetzt werden, welche in ihren nucleophilen Eigenschaften nicht nennenswert mit den Hydroxylamin-Derivaten der Formel (IV) konkurrieren.

Als solche seien Alkalimetall- und Erdalkalimetallcarbonate, wie z.B. Kaliumcarbonat und Calciumcarbonat, tertiäre Amine, wie z.B. Triethylamin, N,N-Dimethylanilin und N,N-Dimethylbenzylamin, sowie Stickstoffheterocyclen wie z.B. Pyridin, Diazabicyclooctan (DABCO) und Diazabicycloundecen (DBU) genannt.

Die Reaktionstemperatur kann bei Verfahren (b) innerhalb eines größeren Bereiches variiert werden. Im allgemeinen arbeitet man zwischen 0°C und 150°C, vorzugsweise zwischen 10°C und 100°C. Verfahren (b) wird im allgemeinen bei Normaldruck durchgeführt.

Zur Durchführung des erfindungsgemäßen Verfahrens (b) setzt man auf 1 Mol Verbindung der Formel (ID) im allgemeinen zwischen 1 und 10 Mol, vorzugsweise zwischen 2 und 5 Mol Hydroxylamin-Derivat der Formel (IV) oder dessen Hydrochlorid ein.

Im allgemeinen wird die Verbindung der Formel (ID) mit dem Verdünnungsmittel bei 20 °C oder leichtem Kühlen vorgelegt und das Hydroxylamin-Derivat der Formel (IV) bzw. das Hydrochlorid hiervon und ein geeigneter Säureakzeptor

wird dazu gegeben. Das Reaktionsgemisch wird dann im allgemeinen bei 20 °C oder erhöhter Temperatur bis zum Reaktionsende gerührt.

Die Aufarbeitung kann nach üblichen Methoden durchgeführt werden. Soweit die Produkte der Formel (I) aus dem Reaktionsgemisch kristallin anfallen, können sie durch Absaugen isoliert werden. Anderenfalls wird - gegebenenfalls nach Einengen - mit Wasser verdünnt und mit einem mit Wasser praktisch nicht mischbaren Lösungsmittel, wie z.B. Methylenchlorid, extrahiert. Durch Waschen der Extraktionslösung mit Wasser, Trocknen, Filtrieren, Einengen des Filtrats und Umkristallisieren des Rückstandes können die Produkte der Formel (I) in reiner Form erhalten werden.

Das erfindungsgemäße Verfahren (c) wird vorzugsweise unter Verwendung von Verdünnungsmitteln durchgeführt. Als solche kommen praktisch alle inerten organischen Lösungsmittel in Betracht. Hierzu gehören insbesondere Alkohole, wie z.B. Ethanol, n- und iso-Propanol, Ether, wie z.B. Tetrahydrofuran, Dioxan und 1,2-Dimethoxyethan, Ester, wie z.B. Essigsäuremethylester und -ethylester sowie Nitrile, wie z.B. Acetonitril.

Die Reaktionstemperatur kann bei Verfahren (c) innerhalb eines größeren Bereiches variiert werden. Im allgemeinen arbeitet man zwischen -20°C und +50°C, vorzugsweise zwischen 0°C und 30°C. Verfahren (c) wird im allgemeinen bei Normaldruck durchgeführt.

Zur Durchführung des erfindungsgemäßen Verfahrens (c) setzt man je Mol Oxyguanidin-Derivat der Formel (I) im allgemeinen zwischen 0,9 und 1,2 Mol, vorzugsweise zwischen 0,95 und 1,1 Mol Metallverbindung ein.

Im allgemeinen werden die Oxyguanidin-Derivate der Formel (I) und das Verdünnungsmittel vorgelegt und - gegebenenfalls unter leichter Außenkühlung - die Metallverbindung - gegebenenfalls im Verdünnungsmittel gelöst - zudosiert. Das Reaktionsgemisch wird bis zum Reaktionsende gerührt. Die salzartigen Produkte der Formel (I) fallen im allgemeinen kristallin an und können durch Absaugen isoliert werden.

Das erfindungsgemäße Verfahren (d) wird vorzugsweise unter Verwendung von Verdünnungsmitteln durchgeführt. Als solche kommen praktisch alle inerten organischen Lösungsmittel in Betracht. Hierzugehören insbesondere Alkohole, wie Methanol, Ethanol, n- und iso-Propanol, Ether, wie Tetrahydrofuran, Dioxan und 1,2-Dimethoxyethan, Ester, wie Essigsäuremethylester und -ethylester sowie Ketone, wie Aceton, Methylethylketon und Methylisobutylketon.

Soweit die als Ausgangsstoffe verwendeten Säuren in wässriger Lösung eingesetzt werden, kann vorteilhaft auch Essigsäureanhydrid als Verdünnungsmittel verwendet werden.

Die Reaktionstemperatur kann bei Verfahren (d) innerhalb eines größeren Bereiches variiert werden. Im allgemeinen

arbeitet man zwischen -20°C und +50°C, vorzugsweise zwischen 0°C und 30°C. Verfahren (d) wird im allgemeinen bei Normaldruck durchgeführt.

Zur Durchführung des erfindungsgemäßen Verfahrens (d) setzt man je Mol Oxyguanidin-Derivat der Formel (I) im allgemeinen zwischen 1 und 10 Mol, vorzugsweise zwischen 1 und 5 Mol einer starken Säure ein.

Im allgemeinen werden die Oxyguanidin-Derivate der Formel (I) und das Verdünnungsmittel vorgelegt und - gegebenenfalls unter leichter Außenkühlung - die starke Säure zudosiert. Das Reaktionsgemisch wird bis zum Reaktionsende gerührt. Die 1 : 1 - Addukte fallen im allgemeinen kristallin an und können durch Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne

Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können zur Bekämpfung monokotyler und dikotyler Unkräuter in monokotylen und dikotylen Kulturen im Vorauflauf- und Nachauflauf-Verfahren eingesetzt werden.

Eine Kontrolle keimender, auflaufender und bereits etablierter Unkräuter in Dauerkulturen ist mit den erfindungsgemäßen Wirkstoffen ebenso möglich, wie die totale Vegetationsbekämpfung auf Nichtkulturland.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. N-(2-Benzthiazolyl)-N,N'-dimethyl-harnstoff, 3-(3-Chlor-4-methylphenyl)-1,1-dimethylharnstoff, 3-(4-isopropylphenyl)-1,1-dimethylharnstoff, 4-Amino-6-(1,1-dimethyl-ethyl)-3-methylthio-1,2,4-triazin-5(4H)-on, 4-Amino-6-(1,1-dimethyl-ethyl)-3-ethylthio-1,2,4-triazin-5(4H)-on, 1-Amino-6-ethylthio-3-(2,2-dimethylpropyl)-1,3,5-triazin-2,4-(1H, 3H)-dion, 4-Amino-3-methyl-6-phenyl-1,2,4-triazin-5(4H)-on, 2-Chlor-4-ethylamino-6-isopropyl-amino-1,3,5-triazin, das R-Enantiomere des 2-[4-(3,5-Dichlor-pyridin-2-oxy)-phenoxy]-propionsäure-(trimethylsilyl)-methylesters, das R-Enantiomere des 2-[4-(3,5-Dichlorpyridyl-2-oxy)-phenoxy]-propionsäure-(2-benzyloxy)-ethylesters, 2,4-Dichlorphenoxyessigsäure, 2-(2,4-Dichlorphenoxy)-propionsäure, 4-Chlor-2-methyl-phenoxy-essigsäure, 2-(2-Methyl-4-chlor-phenoxy)-propionsäure, 3,5-Dijod-4-hydroxy-benzonitril, 3,5-Dibrom-4-hydroxy-benzonitril sowie Diphenylether und Phenylpyridazine, wie z.B. Pyridate. Einige Mischungen zeigen überraschenderweise auch synergistische Wirkung.

Auch eine Mischung mit anderen bekannten Wirkstoffen, wie fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden. Insbesondere bei Anwendung im Nachauflaufverfahren können die erfindungsgemäßen Wirkstoffe auch in Kombination mit emulgierbaren Ölen, oberflächenaktiven Substanzen und anderen Additiven ausgebracht werden.

Die angewandte Wirkstoffmenge kann in einem größeren Bereich schwanken. Sie hängt im wesentlichen von der Art des gewünschten Effekts ab. Im allgemeinen liegen die Aufwandmengen zwischen 0,001 und 10 kg Wirkstoff pro Hektar Bodenfläche, vorzugsweise zwischen 0,05 und 5 kg pro ha.

Die erfindungsgemäßen Wirkstoffe zeigen auch eine fungizide Nebenwirkung, z.B. eine Wirkung gegen Pyricularia oryzae am Reis, sowie eine (systemische) bakterizide Wirksamkeit.

Die Herstellung und die Verwendung der erfindungsgemäßen Wirkstoffe geht aus den nachfolgenden Beispielen hervor.

Herstellungsbeispiele:

Beispiel 1

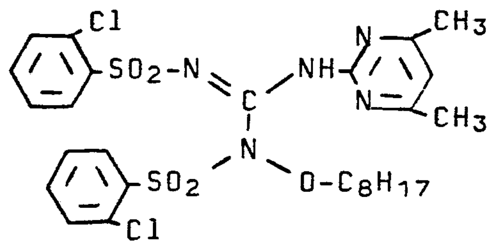

(Verfahren (a))

Eine Mischung aus 7,4 g ( 0,025 Mol ) N'-(4,6-Dimethylpyrimidin-2-yl)-N''-octyloxy-guanidin, 10,7 g (0,051 Mol) 2-Chlor-benzolsulfonsäurechlorid und 30 ml Pyridin wird 15 Stunden bei 20 °C gerührt. Nach weitgehendem Abdestillieren des Pyridins im Wasserstrahlvakuum wird der Rückstand durch Digerieren mit Ethanol zur Kristallisation gebracht und das Produkt wird durch Absaugen isoliert.

Man erhält 7,7 g (48 % der Theorie) N'-(4,6-Dimethylpyrimidin-2-yl)-N''-octyloxy-N'',N'''-bis-(2-chlorbenzolsulfonyl)-guanidin vom Schmelzpunkt 120 °C.

Beispiel 2

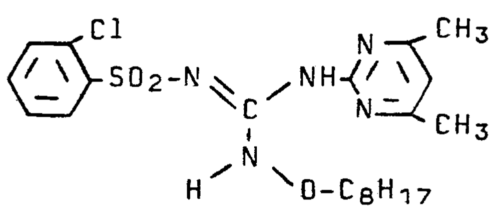

(Verfahren (b))

Eine Mischung aus 13,8 g (0,025 Mol) N'-(4,6-Dimethyl-pyrimidin-2-yl)-N''-methoxy-N'',N'''-bis-(2-chlorbenzol-sulfonyl)-guanidin, 3,7 g (0,026 Mol) O-Octylhydroxylamin und 80 ml Ethanol wird 15 Stunden unter Rückfluß zum Sieden erhitzt. Dann wird im Wasserstrahlvakuum eingeengt, das Produkt durch Anreiben zur Kristallisation gebracht und durch Absaugen isoliert.

Man erhält 3,2 g (27 % der Theorie) N'-(4,6-Dimethylpyrimidin-2-yl)-N''-octyloxy-N'''-(2-chlor-benzolsulfonyl)-guanidin vom Schmelzpunkt 55 °C.

Beispiel 3

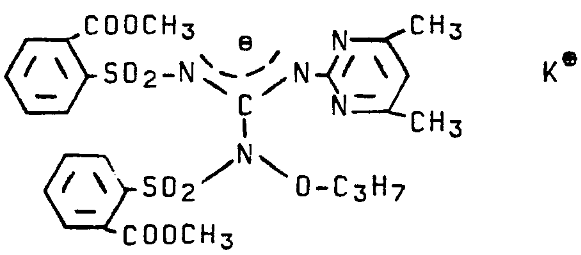

(Verfahren (c))

Eine Mischung aus 6,2 g (0,01 Mol) N'-(4,6-Dimethylpyrimidin-2-yl)-N''-propoxy-N'',N'''-bis-(2-methoxy-carbonyl-benzolsulfonyl)-guanidin, 0,9 g (0,011 Mol) Kaliumethanolat und 20 ml Ethanol wird 15 Stunden bei 20 °C gerührt. Dann wird das kristallin angefallene Produkt durch Absaugen isoliert.

Man erhält 5,4 g (82 % der Theorie) N'-(4,6-Dimethylpyrimidin-2-yl)-N''-propoxy-N'',N'''-bis-(2-methoxy-carbonyl-benzolsulfonyl)-guanidin-Kaliumsalz vom Schmelzpunkt 149 °C.

Beispiel 4

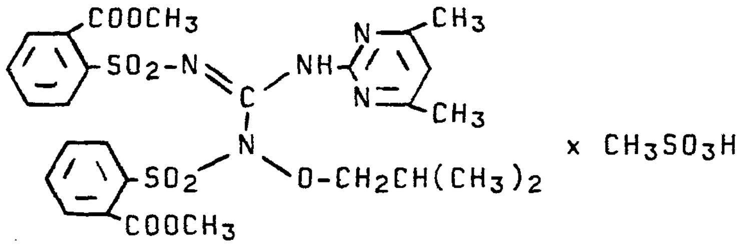

(Verfahren (d))

Eine Mischung aus 6,3 g (0,01 Mol) N'-(4,6-Dimethylpyrimidin-2-yl)-N''-isobutoxy-N'',N'''-bis-(2-methoxycarbonyl-benzolsulfonyl)-guanidin, 1,0 g (0,01 Mol) Methansulfonsäure und 15 ml Aceton wird 24 Stunden bei 20 °C gerührt. Das kristallin angefallene Produkt wird durch Absaugen isoliert.

Man erhält 6,8 g (94 % der Theorie) N'-(4,6-Dimethylpyrimidin-2-yl)-N''-isobutoxy-N'',N'''-bis-(2-methoxycarbonyl-benzolsulfonyl)-guanidin-methansulfonat vom Schmelzpunkt 85 °C.

Nach dem in den vorausgehenden Beispielen beschriebenen Verfahren (a) bis (c) können die in der nachstehenden Tabelle 1 aufgeführten Verbindungen der Formel (I) hergestellt werden:

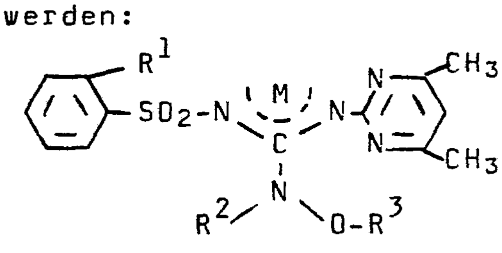

(I)

Tabelle 1

| Beispiel Nr. | $R^1$ | $R^2$ | $R^3$ | M | Schmelzpunkt (°C) |
|---|---|---|---|---|---|
| 5 | Cl | 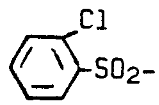 | $-C_4H_9-n$ | H | 120 |
| 6 | $-COOCH_3$ | 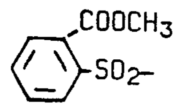 | $-C_4H_9-n$ | H | 115 |
| 7 | $-COOCH_3$ | 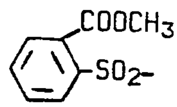 | $-C_8H_{17}-n$ | H | 90 |
| 8 | $-COOCH_3$ | 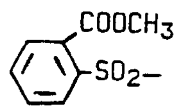 | $-CH_2COOC_2H_5$ | H | 134 |
| 9 | Cl | 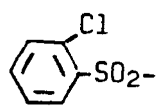 | $-CH_2COOC_2H_5$ | H | 116 |
| 10 | $-COOCH_3$ | 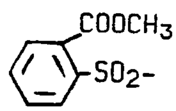 | $-CH_2CH(CH_3)_2$ | H | 147 |
| 11 | Cl | 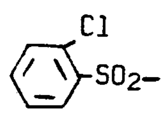 | $-CHCH_2CH_3$<br>$\vert$<br>$CH_3$ | H | 176 |
| 12 | $-COOCH_3$ | 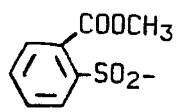 | $-CHCH_2CH_3$<br>$\vert$<br>$CH_3$ | H | 113 |

Tabelle 1 - Fortsetzung

| Beispiel Nr. | $R^1$ | $R^2$ | $R^3$ | M | Schmelzpunkt (°C) |
|---|---|---|---|---|---|
| 13 | Cl | 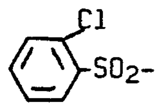 | $-CH_2CH(CH_3)_2$ | H | 131 |
| 14 | $-COOCH_3$ | 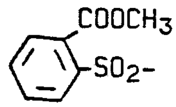 | $-CH_2CH=CH_2$ | H | 102 |
| 15 | $-COOCH_3$ | 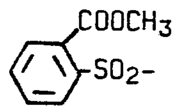 | $-CH_2CH_2CH_3$ | Na | 165 |
| 16 | $-COOCH_3$ | 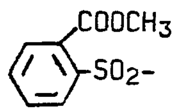 | $-CH_2CH_2CH_3$ | 1/2 Ca | 60 |
| 17 | Cl | 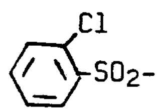 | 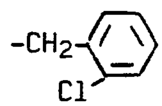 | H | halbkristal-lines Oel |
| 18 | Cl | H | $-CH_2COOCH_3$ | H | 124 |
| 19 | Cl | H | $-CH_2COOC_2H_5$ | H | 110 |
| 20 | Cl | H | $-CH_2COOCH(CH_3)_2$ | H | 113 |
| 21 | Cl | H | $-CH(CH_3)COOCH_3$ | H | 125 |
| 22 | Cl | H | $-CH(CH_3)COOC_2H_5$ | H | 129 |

Tabelle 1 - Fortsetzung

| Beispiel Nr. | $R^1$ | $R^2$ | $R^3$ | M | Schmelzpunkt (°C) |
|---|---|---|---|---|---|
| 23 | $-COOCH_3$ | H | $-CH_2COOH$ | H | 140 |
| 24 | $-COOCH_3$ | H | $-CH_2COOCH_3$ | H | 102 |
| 25 | $-COOCH_3$ | H | $-CH_2COOC_2H_5$ | H | 139 |
| 26 | $-COOCH_3$ | H | $-CH_2COOCH(CH_3)_2$ | H | 83 |
| 27 | $-COOCH_3$ | H | $-CH(CH_3)COOCH_3$ | H | 100 |
| 28 | $-COOCH_3$ | H | $-CH(CH_3)COOC_2H_5$ | H | 56 |
| 29 | Cl | 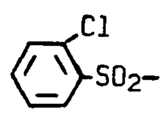 | $-CH_2CH_2-$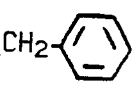 | H | 190 |
| 30 | $-COOCH_3$ | 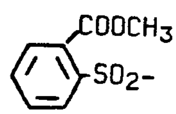 | $-CH_2CH_2-$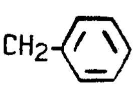 | H | 141 |
| 31 | $-CH_2Cl$ | 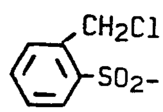 | $-CH_2-CH(CH_3)_2$ | H | 149 |
| 32 | $-CH_2Cl$ | 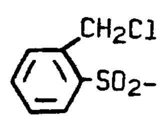 | $-CH(CH_3)-C_2H_5$ | H | 159 |

Tabelle 1 - Fortsetzung

| Beispiel Nr. | $R^1$ | $R^2$ | $R^3$ | M | Schmelzpunkt (°C) |
|---|---|---|---|---|---|
| 33 | $-COOCH_3$ | H | $-CH_2-CH(CH_3)_2$ | H | 110 |
| 34 | $-COOCH_3$ | H | $-\underset{CH_3}{CH}-C_2H_5$ | H | 105 |
| 35 | Cl | H | 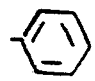 | H | 146 - 149 |
| 36 | $-COOCH_3$ | H | 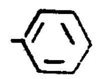 | H | 143 - 145 |
| 37 | $-COOCH_3$ | 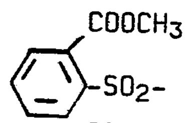 | 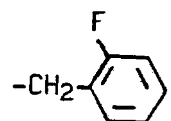 | H | (amorph) |
| 38 | $-COOCH_3$ | 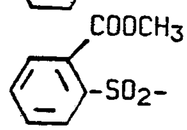 | 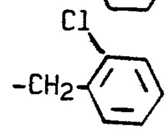 | H | (amorph) |
| 39 | $-COOCH_3$ | 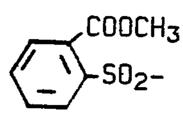 | 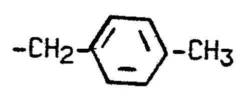 | H | 70 - 71 |
| 40 | $-COOCH_3$ | 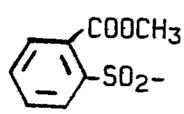 | $-CH(CH_3)_2$ | H | 109 |

Tabelle 1 (Fortsetzung)

| Beispiel Nr. | $R^1$ | $R^2$ | $R^3$ | M | Schmelzpunkt (°C) |
|---|---|---|---|---|---|
| 41 | $-COOCH_3$ | H | 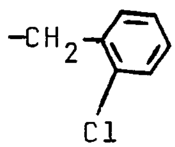 | H | amorph |
| 42 | -Cl | 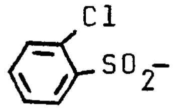 | 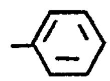 | H | 135-37 |
| 43 | -Cl | 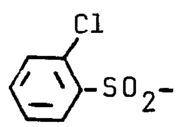 | $-CH(CH_3)_2$ | H | 185 |
| 44 | $-COOCH_3$ | H | $-CH_2CH_2CH_2Cl$ | H | 111 |
| 45 | -Cl | 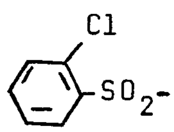 | 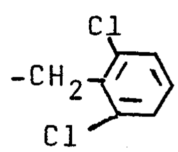 | H | 185 |
| 46 | $-COOCH_3$ | 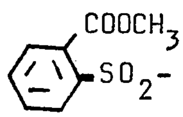 | 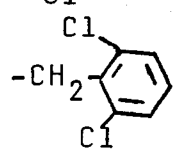 | H | 170 |
| 47 | $-COOCH_3$ | H | 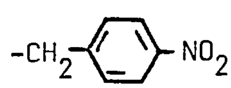 | H | 155 |
| 48 | $-COOCH_3$ | H | 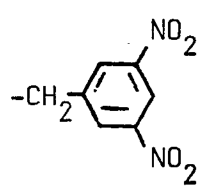 | H | 137 |

Ferner wurden analog Verfahren (d) folgende Säureaddukte von Verbindungen der Formel (I) erhalten:

(12a) 1 : 1-Addukt vom Produkt aus Beipiel (12) mit Methansulfonsäure vom Schmelzpunkt 158 °C,

(12b) 1 : 1-Addukt vom Produkt aus Beispiel (12) mit Schwefelsäure vom Schmelzpunkt 136 °C,

(10a) 1 : 1-Addukt vom Produkt aus Beispiel (10) mit Schwefelsäure als halbfeste Masse,

(31a) 1 : 1-Addukt vom Produkt aus Beipiel (31) mit Methansulfonsäure, vom Schmelzpunkt 169 °C,

(40a) 1 : 1-Addukt vom Produkt aus Beispiel (40) mit Methansulfonsäure, vom Schmelzpunkt 157°C,

(14a) 1 : 1-Addukt vom Produkt aus Beispiel (14) mit Methansulfonsäure, vom Schmelzpunkt 126°C ,

(14b) 1 : 1-Addukt vom Produkt aus Beispiel (14) mit Schwefelsäure, vom Schmelzpunkt 119°C,

(44a) 1:1-Addukt vom Produkt aus Beispiel (44) mit Schwefelsäure, vom Schmelzpunkt 165 °C;

(45a) 1:1-Addukt vom Produkt aus Beispiel(45) mit p-Toluolsulfonsäure, vom Schmelzpunkt 167 °C;

(46a) 1:1-Addukt vom Produkt aus Beispiel (46) mit p-Toluolsulfonsäure, vom Schmelzpunkt 160 °C;

(46b) 1:1-Addukt vom Produkt aus Beispiel (46) nit Ethansulfonsäure, vom Schmelzpunkt 120 °C.

Ausgangsstoffe der Formel (II)

Beispiel (II-1)

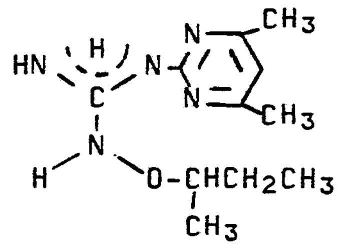

Eine Mischung aus 143 g (0,97 Mol) 2-Cyanamino-4,6-dimethyl-pyrimidin, 94,3 g (1,06 Mol) O-sec.-Butyl-hydroxylamin und 190 ml Ethanol wird 6 Stunden unter Rückfluß zum Sieden erhitzt. Dann wird abgesaugt, das Filtrat eingeengt und der Rückstand mit 500 ml Wasser versetzt. Das hierbei kristallin angefallene Produkt wird durch Absaugen isoliert.

Man erhält 131 g (57 % der Theorie) N'-(4,6-Dimethyl-pyrimidin-2-yl)-N''-sec.-butoxy-guanidin vom Schmelzpunkt 52 °C.

Analog können die in der nachstehenden Tabelle 2 aufgeführten Verbindungen der Formel (II) hergestellt werden:

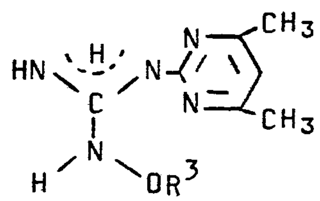

(II)

Tabelle 2

| Beispiel Nr. | $R^3$ | Schmelzpunkt (°C) |
|---|---|---|
| II-2 | $-CH_2CH(CH_3)_2$ | 78 |
| II-3 | $-CH_2CH=CH_2$ | 103 |
| II-4 | $-CH(CH_3)_2$ | 84 |
| II-5 | 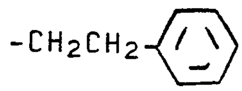 | $n_D^{24}$ : 1,5776 |
| II-6 | $-C_4H_9-n$ | (Oel) |
| II-7 | $-C_8H_{17}-n$ | 58 |
| II-8 | 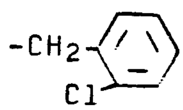 | 102 - 103 |
| II-9 | $-CH_2CH_2CH_2Cl$ | 137 |
| II-10 | 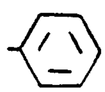 | 192 (Zers.) |
| II-11 | $-CH_2-COOCH_3$ | 148 - 149 |
| II-12 | $-CH_2-COOC_2H_5$ | 98 - 99 |
| II-13 | $-CH-COOCH_3$<br>$\vert$<br>$CH_3$ | 147 - 148 |
| II-14 | 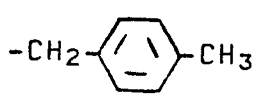 | 85 - 86 |

Tabelle 2 - Fortsetzung

| Beispiel Nr. | $R^3$ | Schmelzpunkt (°C) |
| --- | --- | --- |
| II-15 | 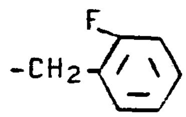 | 114 |
| II-16 | 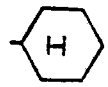 | |
| II-17 | 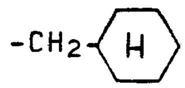 | |
| II-18 | $-CH_2CON(CH_3)_2$ | |
| II-19 | $-CH_2OCH_3$ | |
| II-20 | $-CH_2SCH_3$ | |
| II-21 | $-CH_2CF_3$ | |
| II-22 | 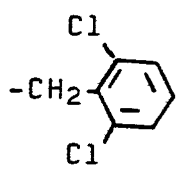 | 145 |
| II-23 | 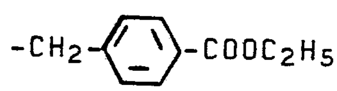 | 138 |
| II-24 | 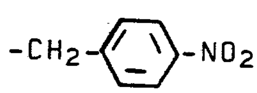 | 172 |
| II-25 | $-CH_2-CH_2-CH_3$ | (Oel) |
| II-26 | $-CH_2-COOC_3H_7-i$ | 112 |
| II-27 | $-CH(C_6H_5)_2$ | |

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Die erfindungsgemäßen Wirkstoffe zeigen in diesem Test eine sehr gute herbizide Wirksamkeit. Dies gilt z.B. in besonderem Maße für die Verbindungen gemäß Herstellungsbeispielen 3, 4, 5, 6, 7, 10, 12, 40.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Die erfindungsgemäßen Wirkstoffe zeigen in diesem Test eine sehr gute herbizide Wirksamkeit. In besonderem Maße gilt dies z.B. für die Verbindungen gemäß Herstellungsbeispielen 1, 3, 4, 5, 6, 7, 10, 12, 14, 40.

Patentansprüche

1) Oxyguanidin-Derivate der allgemeinen Formel (I)

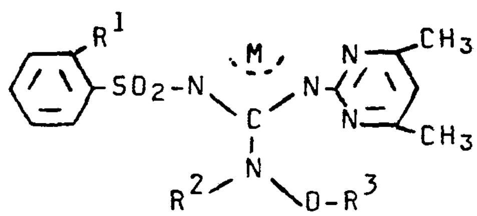

(I)

in welcher

M für Wasserstoff oder ein Metalläquivalent steht,

$R^1$ für Halogen, $C_1$-$C_4$-Alkyl, $C_1$-$C_2$-Halogenalkyl, $C_1$-$C_4$-Alkoxy, Phenyl oder $C_1$-$C_2$-Alkoxy-carbonyl steht,

$R^2$ für Wasserstoff oder für den Rest 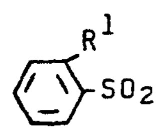 steht, worin $R^1$ die oben angegebene Bedeutung hat, und

$R^3$ für $C_4$-$C_{12}$-Alkyl, Isopropyl oder - für den Fall, daß M für ein Metalläquivalent steht - auch Propyl, für $C_2$-$C_{12}$-Halogenalkyl, für $C_1$-$C_4$-Alkoxy-carbonyl-$C_1$-$C_2$-alkyl, für Aminocarbonylmethyl, $C_1$-$C_4$-Alkyl-amino-carbonylmethyl oder Di-$C_1$-$C_4$-Alkyl-amino-carbonyl-methyl, für $C_3$-$C_{12}$-Alkenyl, für $C_3$-$C_{12}$-Alkinyl, für Carboxy-$C_1$-$C_2$-alkyl, für durch Halogen, Nitro, Cyano, $C_1$-$C_4$-Alkyl, $C_1$-$C_4$-Alkoxy oder $C_1$-$C_4$-Alkoxy-carbonyl substituiertes Benzyl, oder für gegebenenfalls durch

Halogen, Nitro, Cyano, $C_1$-$C_4$-Alkyl, $C_1$-$C_4$-Alkoxy oder $C_1$-$C_4$-Alkoxycarbonyl substituiertes Phenyl, Phenylethyl und Benzhydryl steht,

sowie 1 : 1-Addukte von Verbindungen der Formel (I) mit starken Säuren.

2) Verfahren zur Herstellung von Oxyguanidin-Derivaten der allgemeinen Formel (I) gemäß Anspruch 1, dadurch gekennzeichnet, daß man

(a) für den Fall, daß $R^2$ für den Rest 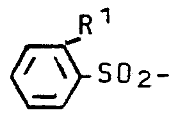 und M für Wasserstoff steht, 4,6-Dimethyl-2-oxyguanidino-pyrimidin-Derivate der Formel (II)

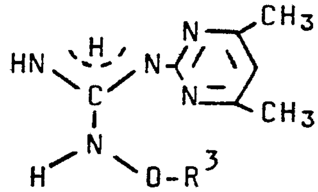

(II)

in welcher

$R^3$ die oben angegeben Bedeutung hat,

mit wenigstens zwei Moläquivalenten von Arensulfonsäurechloriden der Formel (III)

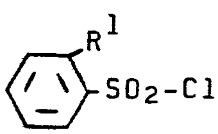

(III)

in welcher

$R^1$ die oben angegebene Bedeutung hat,

in Gegenwart von Säureakzeptoren und gegebenenfalls in Gegenwart von Verdünnungsmitteln umsetzt;

oder daß man

(b) für den Fall, daß $R^2$ für Wasserstoff steht, die nach dem unter (a) angegebenen Verfahren erhältlichen Oxyguanidin-Derivate der Formel (ID)

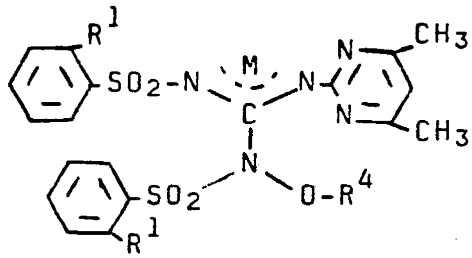

(ID)

in welcher

M und $R^1$ die oben angegebenen Bedeutungen haben und

$R^4$ die oben für $R^3$ angegebene Bedeutung haben kann oder für Methyl oder Ethyl steht,

mit Hydroxylamin-Derivaten der Formel (IV)

$$H_2N\text{-}OR^3 \qquad (IV)$$

in welcher

$R^3$ die oben angegebene Bedeutung hat,

oder mit Hydrochloriden von Verbindungen der Formel (IV),

gegebenenfalls in Gegenwart von Säureakzeptoren und gegebenenfalls in Gegenwart von Verdünnungsmitteln umsetzt;

oder daß man

(c) für den Fall, daß M für ein Metalläquivalent steht, die nach den oben unter (a) und (b) angegebenen Verfahren erhältlichen Verbindungen der Formel (I), in welcher M für Wasserstoff steht und $R^1$, $R^2$ und $R^3$ die oben angegebenen Bedeutungen haben,

mit Metall-hydroxiden, -hydriden oder -alkanolaten oder mit metallorganischen Verbindungen, gegebenenfalls in Gegenwart von Verdünnungsmitteln umsetzt;

oder daß man

(d) für den Fall, daß 1 : 1-Addukte von Verbindungen der Formel (I) mit starken Säuren herzustellen sind,

Oxyguanidin-Derivate der Formel (I), in welcher M für Wasserstoff steht und $R^1$, $R^2$ und $R^3$ die oben angegebenn Bedeutungen haben,

mit starken Säuren, gegebenenfalls in Gegenwart von inerten Verdünnungsmitteln umsetzt.

3) Herbizide Mittel, gekennzeichnet durch einen Gehalt an mindestens einem Oxyguanidin-Derivat der allgemeinen Formel (I) gemäß Anspruch 1.

4) Verwendung von Oxyguanidin-Derivaten der allgemeinen Formel (I) gemäß Anspruch 1 zur Bekämpfung von unerwünschtem Pflanzenwachstum.

5) Verfahren zur Herstellung von herbiziden Mitteln, dadurch gekennzeichnet, daß man Oxyguanidin-Derivate der allgemeinen Formel (I) gemäß Anspruch 1 mit Streckmitteln und/oder oberflächenaktiven Mitteln vermischt.

6) 4,6-Dimethyl-2-oxyguanidino-pyrimidin-Derivate der Formel (II a)

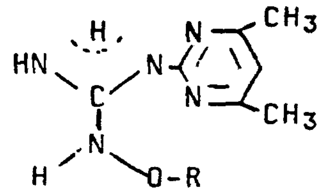

(IIa)

in welcher

R für $C_2$-$C_{12}$-Halogenalkyl, für $C_1$-$C_4$-Alkoxy-carbonyl-$C_1$-$C_2$-alkyl, für Aminocarbonylmethyl, $C_1$-$C_4$-Alkyl-amino-carbonylmethyl oder Di-$C_1$-$C_4$-Alkyl-amino-carbonylmethyl, für $C_4$-$C_{12}$-Alkenyl, für $C_4$-$C_{12}$-Alkinyl, für durch Halogen, Nitro, Cyano, $C_1$-$C_4$-Alkyl, $C_1$-$C_4$-Alkoxy oder $C_1$-$C_4$-Alkoxy-carbonyl substituiertes Benzyl oder für gegebenenfalls durch Halogen, Nitro, Cyano, $C_1$-$C_4$-Alkyl, $C_1$-$C_4$-Alkoxy oder $C_1$-$C_4$-Alkoxy-carbonyl substituiertes Phenyl, Phenylethyl oder Benzylhydryl steht.

7) Verfahren zur Herstellung von 4,6-Dimethyl-2-oxy-guanidino-pyrimidin-Derivaten der Formel (IIa) gemäß Anspruch 6, dadurch gekennzeichnet, daß man 2-Cyanamino-4,6-dimethyl-pyrimidin der Formel (V)

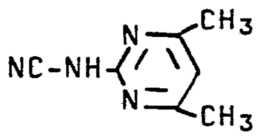

(V)

mit Hydroxylamin-Derivaten der Formel (IVa)

$H_2N-O-R$ (IVa)

in welcher

R die oben angegebenen Bedeutung hat,

oder mit deren Hydrochloriden,

gegebenenfalls in Gegenwart von Verdünnungsmitteln, bei Temperaturen zwischen 20 °C und 150 °C umsetzt und gegebenenfalls die Umsetzungsprodukte mit Säureakzeptoren behandelt.

Europäisches Patentamt

# EUROPÄISCHER RECHERCHENBERICHT

0 173 315

EP 85110826.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | EP - A1 - 0 117 014 (E.I. DU PONT DE NEMOURS)<br>* Zusammenfassung; Formel II; Tabellen 23,25 *<br>-- | 1,3-5 | C 07 D 239/42<br>A 01 N 47/44 |
| P,Y | EP - A1 - 0 136 455 (NIHON TOKUSHU NOYAKU SEIZO)<br>* Zusammenfassung *<br>-- | 1,3-5 | |
| P,Y | EP - A1 - 0 148 498 (NIHON TOKUSHU NOYAKU SEIZO)<br>* Zusammenfassung * | 1,3-5 | |
| P,A | * Seite 2; Formel III *<br>-- | 2,6 | |
| D,A | DD - A - 71 016 (KOCHMANN)<br>* Spalte 1, Zeilen 21-29 *<br>---- | 1,3 | RECHERCHIERTE SACHGEBIETE (Int. Cl.4)<br>C 07 D 239/00<br>A 01 N 47/00 |

Der vorliegende Recherchenbericht wurde für alle Patentansprüche erstellt.

| Recherchenort | Abschlußdatum der Recherche | Prüfer |
|---|---|---|
| WIEN | 22-11-1985 | ONDER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82